# EUROPEAN PATENT SPECIFICATION

(11) **EP 2 512 376 B1**
(45) Date of publication and mention of the grant of the patent: **18.09.2013**
(21) Application number: 10797989.0
(22) Date of filing: 15.12.2010
(51) Int. Cl.: A61F 2/26, A61M 29/00

(54) **INSTRUMENT CONFIGURED TO PREPARE A PENIS FOR IMPLANTATION OF A PENILE PROSTHETIC**
INSTRUMENT ZUR VORBEREITUNG EINES PENIS FÜR DIE IMPLANTATION EINER PENISPROTHESE
INSTRUMENT CONFIGURÉ POUR PRÉPARER UN PÉNIS POUR L'IMPLANTATION D'UNE PROTHÈSE PÉNIENNE

(30) Priority: 15.12.2009 DK 200970276; 16.12.2009 US 639069; 09.07.2010 US 832998
(43) Date of publication of application: 24.10.2012
(73) Proprietor: Coloplast A/S, 3050 Humlebæk (DK)
(72) Inventor: MORNINGSTAR, Randy, L., Brooklyn Park, MN 55444 (US); WOLF, Donald, Shoreview, MN 55126 (US)
(86) International application number: PCT/DK2010/050338
(87) International publication number: WO 2011/072692

(56) References cited:
- WO-A1-03/071970
- WO-A2-02/102230
- US-A- 4 244 370
- US-A- 4 350 151
- US-A- 5 868 729

## Description

### Background

Implanted penile prostheses address erectile dysfunction in men.

In a typical implantation procedure, the penis of the patient is incised in a corporotomy to expose a pair of corpora cavernosa that are aligned axially in a side-by-side orientation within the penis. A cutting implement, such as a curved Mayo scissors, is employed to penetrate the fascia of the penis and form an opening accessing each corpora cavemosum. Thereafter, a tool (e.g., a "Furlow" introducer) is inserted into each corpora cavernosum to measure a length of the penis distally and proximally from a "stay" suture or other stationary landmark locäted near the opening formed in the fascia. Subsequently, each corpora cavernosum is dilated with at least one separate dilation tool, and often multiple dilation tools. For example, each corpora cavernosum is dilated by introducing gradually larger stainless steel rods into the corpora cavernosum to form a recess in the perns that is sized to receive a cylinder of the penile prosthesis.

US4350151 discloses a dilator for use in implant and other types or urologic surgery which reduces tissue abration and damage includes radially separable longitudinal sections for variably dilating generally cylindrical urinary passages and tissue formations. The controlled variable dilation is accomplished by inserting a generally wedge-shaped member between the radially separable sections until the proper degree of dilation has been achieved.

The above-described procedure has proven effective in the implantation of penile prostheses. However, practitioners have expressed a continuing desire for more efficient and cost effective tools and procedures for implanting penile prostheses.

### Summary

One aspect provides an instrument configured to prepare a penis for implantation of a penile prosthetic. The instrument includes a shaft extending between a distal end and a proximal end, a plunger coupled to and movable relative to the shaft, and a dilation head coupled to a distal end of the plunger and disposed around an exterior of the shaft. The dilation head is movable longitudinally along the shaft.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is an exploded perspective view of one embodiment of an instrument that is configured to prepare a penis for implantation of a penile prosthetic.
Figure 2A is a perspective view of one embodiment of a shaft of the instrument illustrated in Figure 1.
Figure 2B is a cross-sectional view of the shaft illustrated in Figure 2A.
Figure 3A is a perspective view of one embodiment of a plunger of the instrument that is insertable into the shaft illustrated in Figure 2A.
Figure 3B is a cross-sectional view of a proximal end portion the plunger illustrated in Figure 3A.
Figure 3C is a cross-sectional view of a distal end portion of the plunger illustrated in Figure 3A.
Figure 4A is a side view of one embodiment of a dilation head that is attachable to the plunger illustrated in Figure 3A.
Figure 4B is an end view of the dilation head illustrated in Figure 4A.
Figure 4C is a cross-sectional view of the dilation head illustrated in Figure 4B.
Figure 5A is a cross-sectional view of the instrument illustrated in Figure 1.
Figure 5B is a cross-sectional view of the instrument illustrated in Figure 5A with the dilation head coupled to a distal end of the plunger.
Figure 6 is an end view of the instrument illustrated in Figure 5B.
Figure 7A is a schematic view of one embodiment of a penis prepared for implantation of a penile prosthetic showing the instrument in a corpora measurement configuration and Figure 7B is a cross-sectional view of the corpora of the penis.
Figures 8A and 8B illustrate the instrument in dilation configurations with the dilation head moved to different locations along the shaft.
Figure 9 is a cross-sectional view of one embodiment of an instrument system that is configured to measure and dilate an opening in a penis.
Figure 10 is a cross-sectional view of one embodiment of an instrument including a dilation head threaded onto a distal end of a plunger.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

The term "proximal" as employed in this application means that the referenced part is situated next to or near the point of attachment or origin or a central point: as located toward a center of the human body. The term "distal" as employed in this application means that the referenced part is situated away from the point of attachment or origin or the central point: as located away from the center of the human body. A distal end is the furthest endmost location of a distal portion of a thing being described, whereas a proximal end is the nearest endmost location of a proximal portion of the thing being described. For example, the glans penis is located distal, and of the crus of the penis is located proximal relative to the male body such that a distal end of a corpora cavernosum of the patient extends about midway into the glans penis.

Multiple different tools and instruments are employed in a typical penile prosthetic implantation procedure to form a recess sized to receive the implant. In general, the fewer tools employed during a body implant procedure, the better.

Embodiments provide an instrument configured to prepare a penis for implantation of a penile prosthetic, where the instrument includes a dilation head that is movable longitudinally along a shaft of the instrument. The dilation head is movable to a proximal end of the shaft such that the shaft has a substantially uniform diameter that is unobstructed and thus configured for measuring a length of the corpora cavernosum. The dilation head is configured to be reversibly movable along the length of the shaft to "core out" and/or dilate the corpora cavernosum. Thus, a single one of the instruments as described herein provides improved dilation of the corpora with improved procedural efficiency by performing the tasks of the multiple different tools and instruments typically employed in a penile prosthetic implantation procedure.

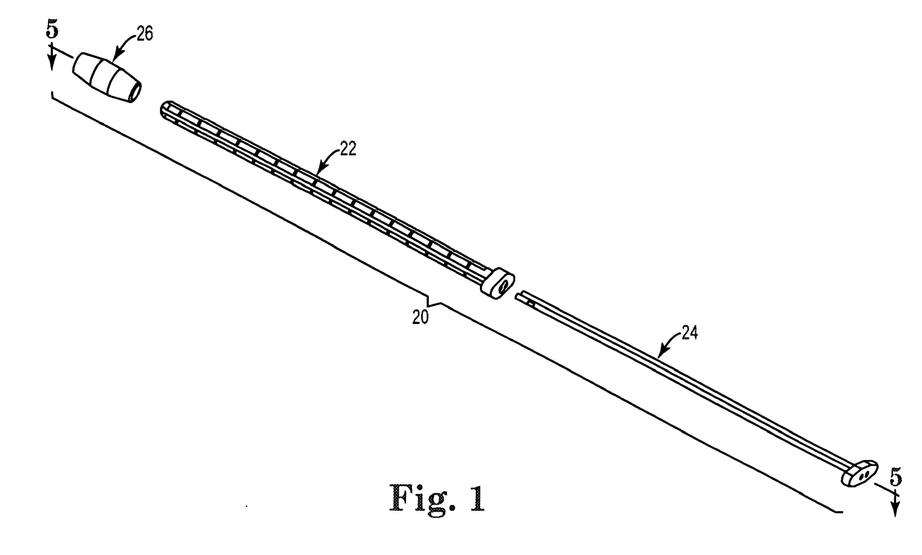

Figure 1 is an exploded perspective view of one embodiment of an instrument 20 that is configured to prepare a penis for implantation of a penile prosthetic. The instrument 20 includes a shaft 22, a plunger 24 that couples with and moves relative to the shaft 22, and a dilation head 26 that couples with the plunger 24. In one embodiment, the plunger 24 is inserted within the shaft 22 and the dilation head 26 is attached to the plunger 24 around the shaft 22. Longitudinal movement of the plunger 24 relative to the shaft 22 moves the dilation head 26 longitudinally along the shaft 22.

During use, as describe below, the plunger 24 is retracted proximally to draw the dilation head 26 toward a proximal end of the shaft 22, which configures the shaft 22 for unobstructed insertion into a corpora cavemosa of the penis to allow the shaft 22 to measure the length of the corpora cavemosa. After the length measurement is taken, and while the shaft 22 is inserted into the corpora cavemosa, the plunger 24 is pushed proximally into the shaft 22 to pass the dilation head 26 along the shaft 22 in the distal direction. The movement of the dilation head 26 along the shaft 22 dilates the tissue in the corpora cavemosa. The dilation head 26 is removable from the shaft 22 and the plunger to allow other differently sized dilation heads 26 to be attached to the plunger 24 for selective dilation of the corpora.

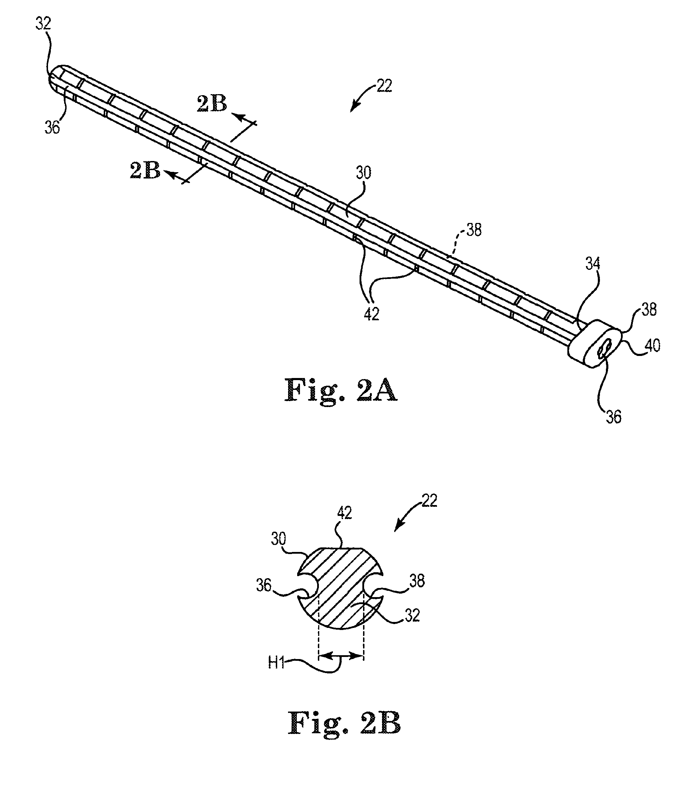

Figure 2A is a perspective view and Figure 2B is a cross-sectional view of one embodiment of the shaft 22. In one embodiment, the shaft 22 has an exterior surface 30 that extends between a distal end 32 and a proximal end 34 and includes a first channel 36 and a second channel 38 that are formed in the exterior surface 30. The exterior surface 30 defines an outside diameter of the shaft 22, and in one embodiment the shaft 22 has a constant outside diameter that is suited for insertion into and measurement of a length of each corpora cavernosum. In one embodiment, the channels 36, 38 are open channels formed from a circular arc of greater than 180 degrees, where the first channel 36 is spaced a first distance H1 apart from the second channel 38.

In one embodiment, the shaft 22 is formed as a single monolithic shaft molded as a unit between the distal end 32 and the proximal end 34, and a handle 40 is attached to the proximal end 34 of the shaft 22.

In one embodiment, at least a portion of the exterior surface 30 of the shaft 22 is provided with indicia 42 placed at selected intervals, for example a series of markings spaced 1 cm apart, although other spacing is also acceptable. In one embodiment, the shaft 22 is fabricated from a circular rod to include the channels 36, 38 and a flat surface on which the indicia 42 are marked. In one embodiment, the indicia 42 are marked on the flat surface of the shaft 22 and over the exterior surface 30. Marking the indicia 42 on the flat surface minimizes the deleterious effects of glare, which can occur in the typically brightly-lighted operating rooms.

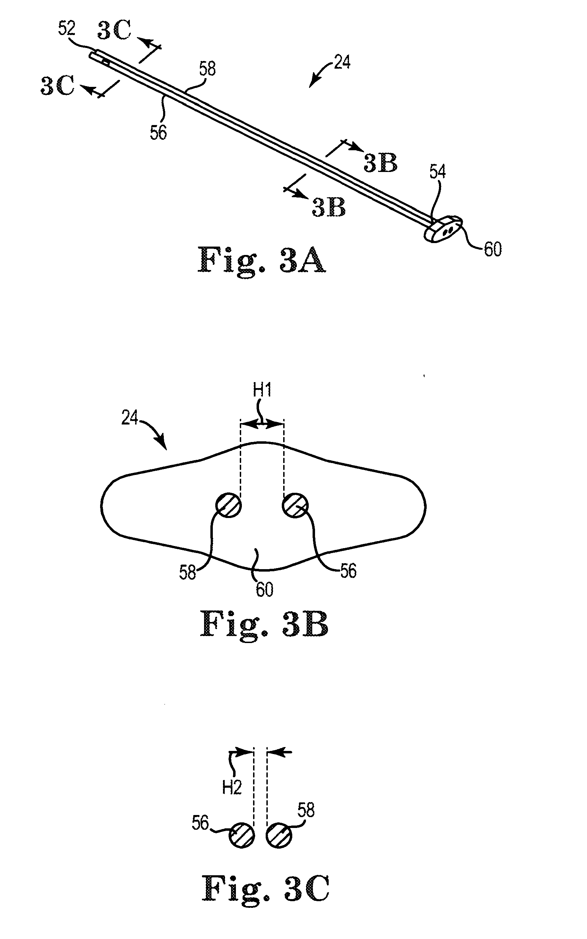

Figure 3A is a perspective view and Figures 3B and 3C are cross-sectional views of one embodiment of the plunger 24. In one embodiment, the plunger 24 extends between a distal end 52 and a proximal end 54 and includes a first rod 56 and a second rod 58 spaced apart from the first rod 56, where the rods 56, 58 extend from a handle 60.

The rods 56, 58 are sized to slide within the channels 36, 38 (Figure 2B). For example, the rods 56, 58 are formed to have a diameter that is similar to or slightly less than the diameter of the channels 36, 38 (in order to allow for clearance between the rods 56, 58 and the channels 36, 38). In one embodiment, the first channel 36 and the second channel 38 are open channels such that at least a portion of the first rod 56 and the second rod 58 is visibly exposed through the exterior surface 30 of the shaft 22 (Figure 2B) when the instrument 20 is assembled.

In one embodiment, the rods 56, 58 are flexible and pre-flexed or stressed such that the distal ends 52 deflect inwardly toward each other as illustrated by the distance H2 in Figure 3C showing the distal ends 52 are more narrowly spaced apart than the distance H1 between the rods 56, 58 near the handle 60 (Figure 3B). In particular, in one embodiment the rods 56, 58 are spaced by the first distance H1 apart near the handle 60 as illustrated in the cross-sectional view of the proximal end portion of Figure 3B and the distal ends 52 of the rods 56, 58 are tensioned (e.g., bent) to flex together such they are spaced apart by a distance H2 that is less than the distance H1 as illustrated in the cross-sectional view of the distal end portion of Figure 3C. In this manner, when the rods 56, 58 are engaged with the channels 36, 38 (Figure 2B) the shaft 22 maintains the rods 56, 58 apart, one from the other, by the distance H1. Alternatively, when the distal ends 52 of the rods 56, 58 extend beyond the distal end 32 of the shaft 22 (Figure 2A) the ends 52 are unconstrained and thus flex and pinch together a distance H2 apart.

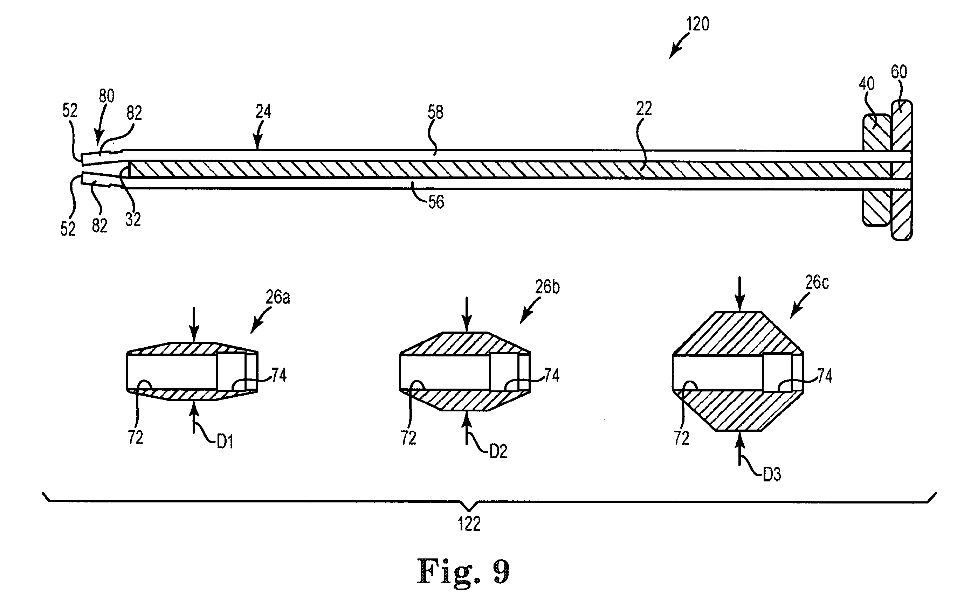

In one embodiment, the rods 56, 58 are formed to be substantially parallel with each other, for example as illustrated by the rods of plunger 134 in Figure 9.

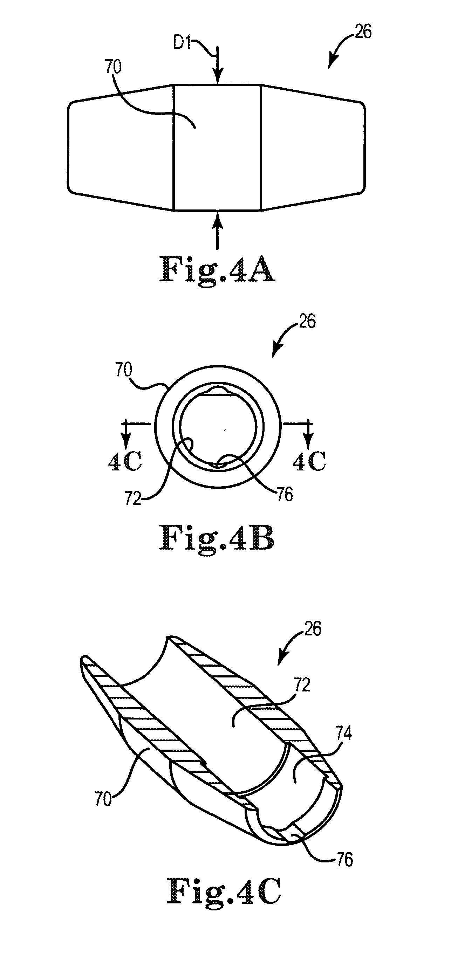

Figure 4A is a side view, Figure 4B is an end view, and Figure 4C is a cross-sectional view of the dilation head 26. In one embodiment, the dilation head 26 includes an exterior surface 70 having an outside diameter of D1 and an inside surface 72 that is formed to provide a recess 74. The dilation head 26 is attachable to the distal ends 52 of the rods 56, 58 and its annular conformation is configured to be disposed over the shaft 22. In one embodiment, the recess 74 is provided as an annular groove formed around the inside surface 72 of the dilation head 26.

In one embodiment, the diameter D1 is larger than the diameter of the shaft 22 (Figure 2B) and the inside surface 72 ofthe dilation head 26 includes clearance notches 76 sized to move over the rods 56, 58 (Figure 3B) of the assembled instrument 20. In one embodiment, the dilation head 26 is removably attachable and re-attachable to the plunger 24 (Figure 3A). Suitable examples of mechanisms that allow the dilation head to be removed from the plunger 24 include tension latches, threads, snap fits, friction fits, and quarter-turn quick attachment mechanisms (e.g., post-and-slot arrangements).

In one embodiment, the instrument 20 is configured to be reusable and is fabricated from a suitable material such as a polymer. Suitable polymers include polysulfone, polyetherimide, or polyester, or blends or derivatives of polysulfone, polyetherimide, or polyester. In one example, the shaft 22, the plunger 24, and the dilation head 26 are each fabricated from polysulfone and thus configured for disposable single surgical use.

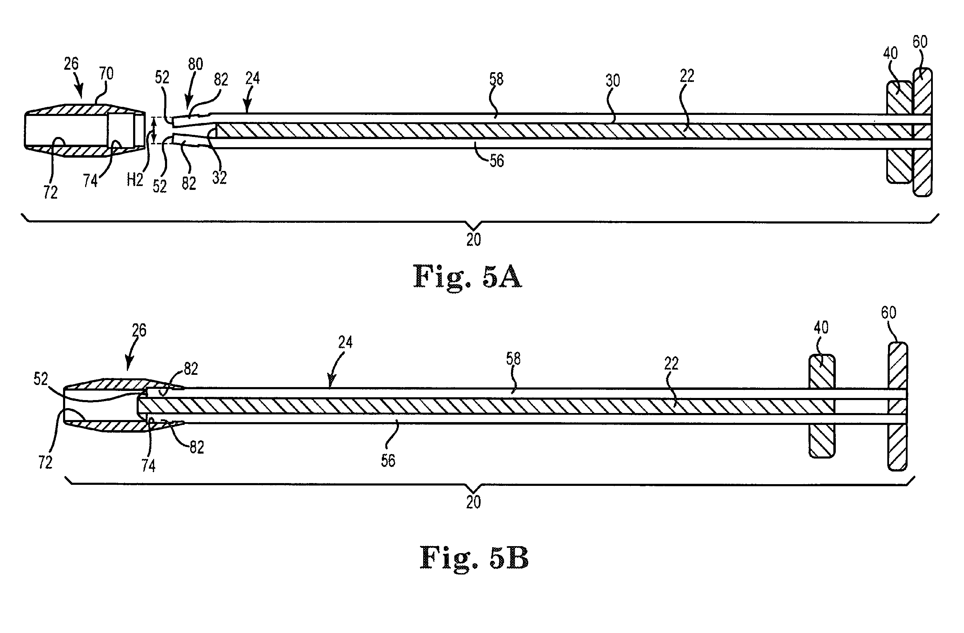

Figure 5A is a cross-sectional view of the dilation head 26 disengaged from the plunger 24. In one embodiment, the plunger 24 is longer than the shaft 22 such that the distal ends 52 of the rods 56, 58 extend beyond the distal end 32 ofthe shaft 22 when the handle 60 ofthe plunger 24 is in contact with a handle 40 ofthe shaft 22. In one embodiment, the distal ends 52 of the rods 56, 58 combine to provide a tension latch 80. The tension latch 80 is flexible and compresses to release from the dilation head 26 (Figure 5A) and expands to engage with the dilation at 26 (Figure 5B).

For example, in one embodiment the tension latch 80 includes a boss 82 that extends from an exterior surface of the each of the rods 56, 58. The tension latch 80 is characterized in that the distal ends 52 ofthe rods 56, 58 deflect inwardly together when the ends 52 of the plunger 24 extend beyond the shaft 22, which provides clearance for the bosses 82 to pass inside the interior surface 72 of the dilation at 26 for attachment/removal of head 26 from plunger 24. For example, and with additional reference to Figure 3C, the distal ends 52 of the rods 56, 58 deflect inwardly to a spacing of approximately H2, which provides clearance for the bosses 82 to enter inside the interior surface 72 of the dilation head 26. That is to say, the inside diameter of the inside surface 72 is about equal to the distance H1 plus twice the diameter of one of the proximal portion of the rods 56, 58.

Figure 5B is a cross-sectional view ofthe dilation head 26 attached to and engaged with the plunger 24. When the dilation head 26 is placed over the compressed distal ends 52 of the rods 56, 58 (Figure 5A) and the plunger 24 is retracted such that the ends 52 no longer extend beyond the shaft 22, then the bosses 82 of the tension latch 80 are forced outward to engage with the recess 74 ofthe dilation head 26. In this manner, a removable dilation head 26 is provided for the instrument 20, which allows the surgeon to select differently sized dilation heads 26 to selectively dilate the corpora of the penis to a desired diameter, as described below.

The plunger 24 is movable longitudinally relative to the shaft 22. Movement of the plunger 24 back and forth relative to the shaft 22 moves the dilation head 26 longitudinally back and forth along the shaft 22. In one embodiment, the handle 40 attached to the shaft 22 is separated from the handle 60 attached to the plunger 24 when the distal ends 52 of the plunger 24 are located between the distal end 32 and a proximal end 30 of the shaft 22.

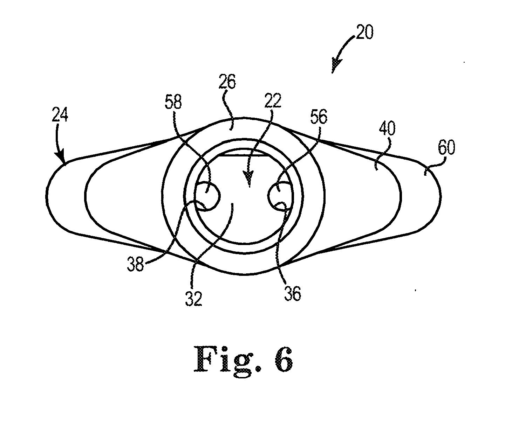

Figure 6 is an end view of the instrument 20. The plunger 24 is engaged with the shaft and 22 such that the rods 56, 58 are engaged with the channels 36, 38 formed in the shaft 22. The dilation head 26 is attached to the rods 56, 58 of the plunger 24 such that the dilation head 26 is disposed around the shaft 22.

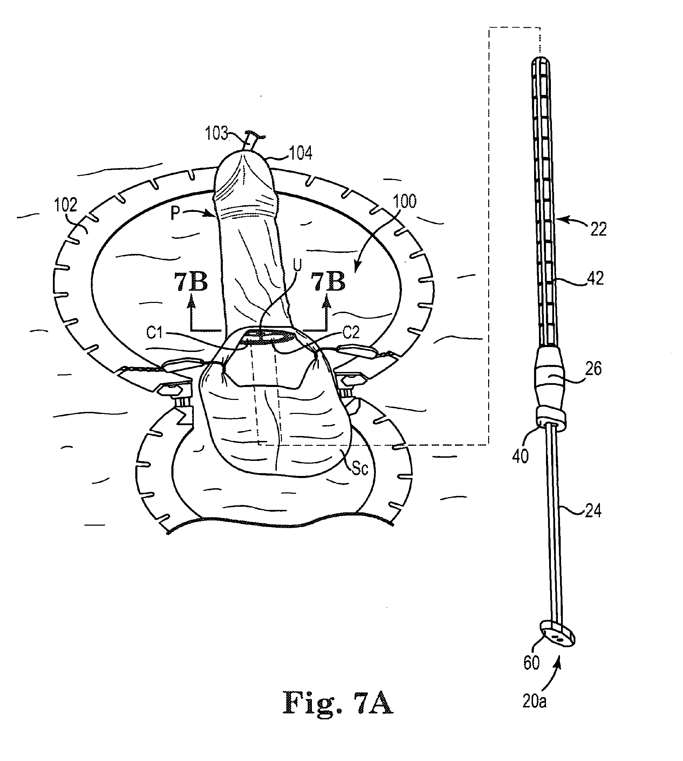

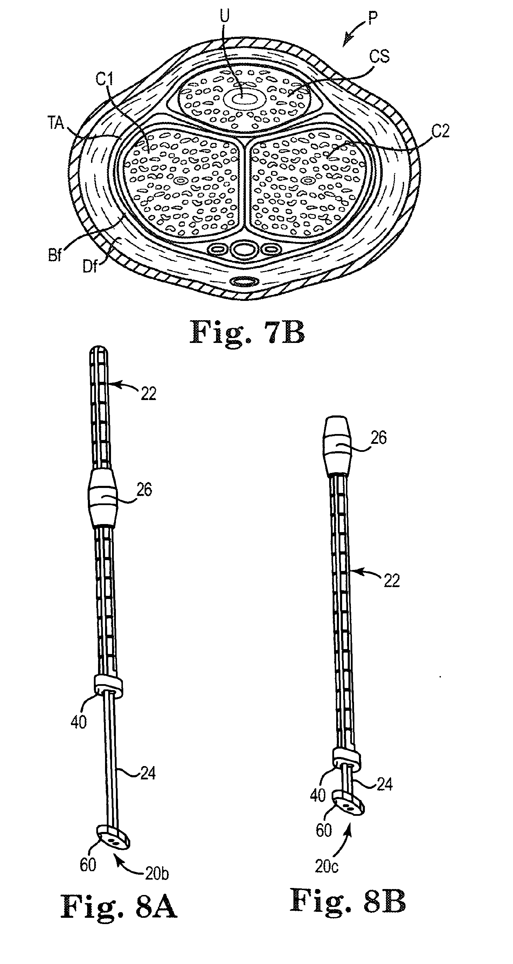

Figure 7A is a schematic view of one embodiment of a penis P prepared for implantation of a penile prosthetic showing the instrument 20a in a corpora measurement configuration and Figure 7B is a cross-sectional view of the corpora C1 and C2 of the penis P. While the penile prosthetic is not shown, it would typically include a pair of inflatable cylinders, a reservoir, and a pump employed to transfer fluid to/from the reservoir, where the instrument 20 is employed to dilate the corpora for insertion of the cylinders.

The penis P is reclined against the torso such that the urethra U, surrounded by corpus spongiosum tissue, is oriented upward. The penis P has been incised to expose the corpora cavemosa (C1 and C2) and the instrument 20a has the dilation head 26 fully retracted proximally to allow the shaft 22 to measure the length of each of the corpora cavernosum (C1 or C2). In the corpora measurement configuration, the entire distal portion of the shaft 22 is unobstructed from the dilation head 26.

The groin area 100 of the patient is shaved, cleaned and suitably prepped with a surgical solution prior to draping with a sterile drape as directed by the healthcare provider's procedures. A retraction device, such as a retractor 102 sold under the trademark Lone Star and available from Lone Star Medical Products of Stafford, TX, is placed around the penis P if so desired by the surgeon to establish a surgically clean field. A catheter 103 is inserted into the urethra U from the distal end 104 of the penis P. Thereafter, the surgeon forms an incision to access the corpora cavernosa C1 and C2 of the penis.

Suitable examples of incisions include either an infrapubic incision or a transverse scrotal incision. The infrapubic incision is initiated between the umbilicus and the penis (i.e., above the penis), whereas the transverse scrotal incision is made across an upper portion of the patient's scrotum Sc.

As an example of the transverse scrotal approach, with reference to Figure 7B, the surgeon forms a 2-3 cm transverse incision through the subcutaneous tissue of the median raphe of the upper scrotum Sc and dissects down through the Darto's fascia Df and Buck's fascia Bf to expose the tunicae albuginea TA of the penis P. Thereafter, each corpora cavernosum C1 and C2 is exposed in a corporotomy where a small (approximately 1.5 cm) incision is formed to allow the surgeon to access and subsequently dilate the corpora cavernosa C1 and C2.

With reference to both Figures 7A and 7B, the surgeon typically will insert a blunt-ended scissors or other elongated tool to separate a portion of the spongiosum material to open a pathway for the instrument 20a. The surgeon inserts the shaft 22 (instrument 20a) into the corpora cavernosa C1 and C2 to measure the proximal and distal length of each corpora cavernosum C1 and C2. For example, the shaft 22 is inserted into one of the corpora cavernosa C1 or C2 forward in the distal penis toward the glans penis, the distal measurement is recorded by reading the indicia 42, and the shaft 22 is inserted into the same corpora cavernosa C1 or C2 rearward in the proximal penis toward the crus of the penis to record the proximal length of the corpora by reading the indicia 42. The distal and proximal measurements would typically be made in reference to a "stay stitch" temporarily placed in the incision. The sum of the distal and the proximal measurements represent the length of the corpora into which the implant is placed. This procedure is repeated for the other of the corpora cavernosa C1 or C2 to measure the length of the companion corpora. Thereafter, each corpora cavernosum C1 and C2 is dilated distally and proximally with the instrument 20.

Figures 8A and 8B illustrate the instrument 20 in the dilation configurations with the dilation head 26 moved midway along the shaft 22 (Figure 8A) and the dilation head 26 moved to the distal end of the shaft (Figure 8B). In one exemplary approach, the surgeon begins dilation of the distal and proximal corpora cavernosum C1 and C2 by introducing a dilation head 26 having an 8 mm outside diameter (D1 in Figure 4A) into the spongy tissue of one of the corpora C1 or C2. The plunger 24 is moved into the shaft 22 to move the dilation head 26 from a proximal location (instrument 20a in Figure 7A) to a distal location (instrument 20c) to open the spongy tissue of the corpora along the length of the penis P. The plunger 24 is withdrawn proximally with the shaft 22 remaining in the penis P, and if the surgeon determines it to be desirable, once again advances the dilation head 26 distally and longitudinally along the shaft 22 to fully dilate the tissue of the corpora. Thereafter, the surgeon may optionally remove the instrument 20 from the penis P, remove the 8 mm dilation head, for example, and attach a larger diameter dilation head 26 to the plunger 24, and insert the newly configured tool 20 into the penis P to sequentially dilate the corpora cavernosum C1 and C2 to a width that accommodates the selected cylinder diameter of the implant.

In another exemplary approach, the surgeon may choose to dilate the distal and proximal corpora by a single introduction of a dilation head having a 14 mm outside diameter. Alternatively, the surgeon may choose to sequentially dilate the corpora with a series of dilation heads 26 having an outside diameter ranging from 8 mm to 10 mm to 12 mm and outward to 14 mm in diameter, in the manner described above. Other dilation heads 26 wider than 14 mm are also within the scope of this disclosure. In any regard, the dilation head 26 is introduced and pushed distally toward the glans penis and proximally toward the crus of the penis to dilate each of the corpora cavernosum C1 and C2 along its length. After dilation of the corpora cavernosum C1 and C2, the surgeon selects a proper length of implant and proceeds with placement of cylinders of the implant within the fully dilated corpora.

Figure 9 is a cross-sectional view of an instrument system 120 including a set 122 of dilation heads 26, each removably attachable to the tension latch 80 of the plunger 24. In one embodiment, the set 122 of dilation heads includes multiple dilation heads 26a, 26b, 26c each having a different diameter, where each dilation head 26a, 26b, 26c is configured to couple to the distal ends 52 of the plunger 24. In one embodiment, the instrument 20 (Figure 1) and the set 122 of dilation heads are provided as a kit of parts.

In one embodiment, dilation head 26a is provided with an outside diameter D1, dilation head 26b is provided with an outside diameter D2, and dilation head 26c is provided with an outside diameter D3, where D3 is greater than D2, and D2 is greater than D1. As an example, in one embodiment the diameter D1 is about 8 mm, the diameter D2 is about 10 mm, and a diameter D3 is about 12 mm. It is to be understood that the set 122 of dilation heads 26 could be provided with diameters ranging from 6 mm to 18 mm or more, in increments of about 2 mm, for example, depending upon the patient size or surgeon preference.

Each of the dilation heads 26a, 26b, 26c is provided with a recess 74 that is configured to couple with the bosses 82 of the tension latch 80. During use, the surgeon would initially measure the length of the corpora cavemosa C1 and C2 with the shaft 22, dilate the distal and proximal corpora cavemosa C1 and C2 (Figure 7B) in the range of D1 with the dilation head 26a, remove the dilation head 26a and attach the larger diameter D2 of the dilation head 26b to the plunger 24, dilate the distal and proximal corpora cavemosa C1 and C2 in the range of D2, and remove the dilation head 26b and attach the even larger diameter of the dilation head 26c to the plunger 24 to sequentially and fully dilate the distal and proximal corpora cavemosa C1 and C2 of the penis P in the range of D3.

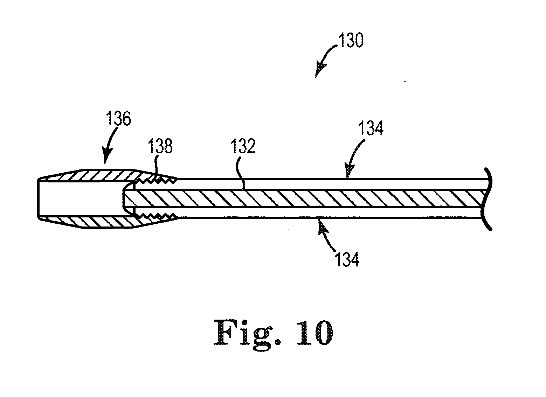

Figure 10 is a cross-sectional view of one embodiment of an instrument 130 that is configured to prepare a penis for implantation of a penile prosthetic and including a dilation head 136 threaded onto a distal end of a plunger 134. The instrument 130 includes a shaft 132 that supports the plunger 134 in a manner similar to that described above for instrument 20. In one embodiment, the distal end of the plunger 134 is threaded to receive threads formed on an inside surface of the dilation head 136. In this manner, the dilation head 136 is removable from the distal end of the plunger 134 in a twist-on and twist-off approach. As described above in Figure 8, one embodiment of instrument 130 includes a set of dilation heads, where each dilation head is removably attachable to the plunger 134 to allow the surgeon to sequentially dilate the corpora cavemosa to an increasingly larger diameter.

Embodiments provide an instrument that is configured to prepare a penis for implantation of a penile prosthetic, where the instrument includes a shaft suited for measuring a length of the corpora cavernosum and a dilation head that is movable longitudinally along the shaft of the instrument to dilate the corpora cavernosum. Thus, a single instrument is provided that has improved cost effectiveness and procedural efficiency over the prior tools, and is suited to measure and dilate the corpora cavernosum of the penis.

## Claims

1. An instrument (20) configured to prepare a penis for implantation of a penile prosthetic, the instrument (20) comprising:
a shaft (22) extending between a distal end (32) and a proximal end (34);
a plunger (24) coupled to and movable relative to the shaft (22); and
a dilation head (26) coupled to a distal end (52) of the plunger (24) and disposed around an exterior (30) of the shaft **characterised in that**, the dilation head (26) is movable longitudinally along the shaft (22).

2. The instrument of claim 1, wherein the plunger (24) comprises a first rod (56) and a second rod (58) and an exterior surface (30) of the shaft (22) defines a first channel (36) sized to receive the first rod (56) and a second channel (38) sized to receive the second rod (58).

3. The instrument of claim 2, wherein the first channel (36) and the second channel (38) are open channels such that at least a portion of the first rod (56) and the second rod (58) is exposed through the exterior surface (30) of the shaft (22).

4. The instrument of claim 2 or 3, wherein the first channel (36) is spaced a first distance (H1) from the second channel (38) such that the first and second rods (56,58) are spaced apart by the first distance (H1) when engaged in a respective one of the first and second channels (36,38).

5. The instrument of claim 4, wherein distal ends (52) of the first and second rods (56,58) flex laterally such that each is spaced apart from the other by a distance (H2) that is less than the first distance (H1) when the distal end (52) of the plunger (24) is moved past the distal end (32) of the shaft (22).

6. The instrument of claim 5, wherein the distal ends (52) of the first and second rods (56,58) provide a tension latch (80), the tension latch (80) engaged with the dilation head (26) when the distal end (52) of the plunger (24) is between the distal end (32) and the proximal end (34) of the shaft (22) and the tension latch (80) disengaged with the dilation head (26) when the distal end (52) of the plunger (24) is moved past the distal end (32) of the shaft (22).

7. The instrument of any one of the claims 1 - 6, wherein the plunger (24) is longer than the shaft (22) and the dilation head (26) is removably coupled to a distal end (52) of the plunger (24).

8. The instrument of claim 7, further comprising:
a first handle (40) attached to the proximal end (32) of the shaft (22); and
a second handle (60) attached to a proximal end (52) of the plunger (24).

9. The instrument of claim 8, wherein the first handle (40) is separated from the second handle (60) when the distal end (52) of the plunger (24) is between the distal end (32) and the proximal end (34) of the shaft (22) and the first handle (40) contacts the second handle (60) when the distal end (52) of the plunger (24) is moved past the distal end (32) of the shaft (22).

10. The instrument of any one of the claims 1 - 9, wherein the dilation head (26) is reversibly movable longitudinally between the distal end (32) of the shaft (22) and the proximal end (34) of the shaft (22).

11. The instrument of any one of the claims 1 - 10, wherein an exterior surface (30) of the shaft (22) comprises measurement indicia (42).

12. The instrument of any one of the claims 1 - 11, wherein the shaft (22) has an outside diameter and the dilation head (26) has a first diameter (D1) that is greater than the outside diameter of the shaft (22).

13. The instrument of any one of the claims 1 - 12, wherein the shaft (22) has a constant outside diameter.

14. The instrument of any one of the claims 1 - 13, wherein the dilation head (26) is a first dilation head that is removable from the plunger (24) and replaceable with a second dilation head having a second diameter that is different from the first diameter of the first dilation head.

15. An instrument system configured to prepare a penis for reception of a penile prosthetic, the instrument system comprising an instrument (20) according to claim 1 and further **characterised in**
a longitudinal shaft (22) having a constant outside diameter configured to measure the opening in a penis;
a plunger (24) coupled to and movable longitudinally within the shaft (22); and
a set (122) of dilation heads (26a,26b,26c), each dilation head configured to couple to a distal end (52) of the plunger (24), surround an exterior (30) of the shaft (22), and move longitudinally along the shaft (22);
wherein each dilation head (26a,26b,26c) in the set (122) of dilation heads has a different outside diameter.

16. The instrument system of claim 15, wherein the plunger (24) comprises two longitudinally disposed rods (56,58) that are separated laterally apart by the shaft (22) to apply engagement tension to each dilation head coupled to the distal end (52) of the plunger (24).

17. The instrument system of claim 16, wherein each rod (56,58) comprises a boss (82) that projects from an exterior surface of the rod (56,58), and each dilation head includes a recess (74) formed on an interior surface of the dilation head, the boss (82) configured to couple with the recess (74).

18. The instrument system of claim 17, wherein the rods (56,58) are movable laterally to disengage the boss (82) from the recess (74).

19. The instrument system of claim 18, wherein the plunger (24) is longer than the shaft (22) such that the rods (56,58) move laterally together when a distal end (52) of the plunger (24) extends over a distal end (32) of the shaft (22).

20. The instrument of claim 1, further comprising:
means for removing the dilation head from the distal end of the plunger.

21. The instrument of claim 1, wherein the shaft (22) comprises a single monolithic shaft.

## Patentansprüche

1. Instrument (20) zur Vorbereitung eines Penis für die Implantation einer Penisprothese, worin das Instrument (20) Folgendes umfasst:
einen sich zwischen einem distalen Ende (32) und einem proximalen Ende (34) erstreckenden Schaft (22);
einen mit dem Schaft (22) verbundenen und relativ dazu beweglichen Kolben (24); und
einen mit einem distalen Ende (52) des Kolbens (24) gekoppelten und um eine Außenseite (30) des Schaftes angeordneten Dilatationskopf (26), **dadurch gekennzeichnet, dass** der Dilatationskopf (26) in Längsrichtung über den Schaft (22) bewegbar ist.

2. Instrument nach Anspruch 1, worin der Kolben (24) eine erste Stange (56) und eine zweite Stange (58) umfasst und eine Außenseite (30) des Schaftes (22) einen ersten Kanal (36) mit einer zur Aufnahme der ersten Stange (56) ausgelegten Größe und einen zweiten Kanal (38) mit einer zur Aufnahme der zweiten Stange (58) ausgelegten Größe definiert.

3. Instrument nach Anspruch 2, worin der erste Kanal (36) und der zweite Kanal (38) offene Kanäle sind, so dass zumindest ein Teil der ersten Stange (56) und der zweiten Stange (58) durch die Außenseite (30) des Schaftes (22) freigelegt ist.

4. Instrument nach Anspruch 2 oder 3, worin der erste Kanal (36) in einem ersten Abstand (H1) von dem zweiten Kanal (38) angeordnet ist, so dass die ersten und zweiten Stangen (56, 58) um den ersten Abstand (H1) voneinander beabstandet sind, wenn sie jeweils mit einem der ersten und zweiten Kanäle (36, 38) in Eingriff stehen.

5. Instrument nach Anspruch 4, worin sich distale Enden (52) der ersten und zweiten Stangen (56, 58) seitlich biegen, so dass jede von der anderen um einen Abstand (H2), der kleiner als der erste Abstand (H1) ist, beabstandet ist, wenn das distale Ende (52) des Kolbens (24) über das distale Ende (32) des Schaftes (22) hinaus bewegt wird.

6. Instrument nach Anspruch 5, worin die distalen Enden (52) der ersten und zweiten Stangen (56, 58) einen Spannriegel (80) bereitstellen, wobei der Spannriegel (80) mit dem Dilatationskopf (26) in Eingriff steht, wenn sich das distale Ende (52) des Kolbens (24) zwischen dem distalen Ende (32) und dem proximalen Ende (34) des Schaftes (22) befindet und der Spannriegel (80) nicht mit dem Dilatationskopf (26) in Eingriff steht, wenn das distale Ende (52) des Kolbens (24) am distalen Ende (32) des Schaftes (22) vorbei bewegt wird.

7. Instrument nach einem der Ansprüche 1-6, worin der Kolben (24) länger als der Schaft (22) ist und der Dilatationskopf (26) entfernbar mit einem distalen Ende (52) des Kolbens (24) gekoppelt ist.

8. Instrument nach Anspruch 7, ferner umfassend:
einen ersten Handgriff (40), der an dem proximalen Ende (32) des Schaftes (22) befestigt ist;
und
einen zweiten Handgriff (60), der an einem proximalen Ende (52) des Kolbens (24) befestigt ist.

9. Instrument nach Anspruch 8 worin der erste Handgriff (40) vom zweiten Handgriff (60) beabstandet ist, wenn sich das distale Ende (52) des Kolbens (24) zwischen dem distalen Ende (32) und dem proximalen Ende (34) des Schaftes (22) befindet und der erste Handgriff (40) mit dem zweiten Handgriff (60) in Berührung steht, wenn das distale Ende (52) des Kolbens (24) an dem distalen Ende (32) des Schaftes (22) vorbei bewegt wird.

10. Instrument nach einem der Ansprüche 1-9, worin der Dilatationskopf (26) reversibel in Längsrichtung zwischen dem distalen Ende (32) des Schaftes (22) und dem proximalen Ende (34) des Schaftes (22) bewegt werden kann.

11. Instrument nach einem der Ansprüche 1-10, worin eine Außenseite (30) des Schaftes (22) Messmarkierungen (42) umfasst.

12. Instrument nach einem der Ansprüche 1-11, worin der Schaft (22) einen Außendurchmesser aufweist und der Dilatationskopf (26) einen ersten Durchmesser (D1) aufweist, der größer als der Außendurchmesser des Schaftes (22) ist.

13. Instrument nach einem der Ansprüche 1-12, worin der Schaft (22) einen konstanten Außendurchmesser aufweist.

14. Instrument nach einem der Ansprüche 1-13, worin der Dilatationskopf (26) ein erster Dilatationskopf ist, der von Kolben (24) entfernt und durch einen zweiten Dilatationskopf mit einem zweiten Durchmesser, der sich vom ersten Durchmesser des ersten Dilatationskopfes unterscheidet, ersetzt werden kann.

15. Instrumentensystem zur Vorbereitung eines Penis für die Aufnahme einer Penisprothese; worin das Instrumentensystem ein Instrument (20) nach Anspruch 1 umfasst und ferner **gekennzeichnet ist durch**:
einen Längsschaft (22) mit einem konstanten Außendurchmesser zur Messung der Öffnung in einem Penis;
einen Kolben (24), der mit dem Schaft (22) verbunden ist und in Längsrichtung in dem Schaft (22) bewegt werden kann; und
einen Satz (122) von Dilatationsköpfen (26a, 26b, 26c), wobei jeder Dilatationskopf zur Verbindung mit einem distalen Ende (52) des Kolbens (24), zur Umgebung einer Außenseite (30) des Schaftes (22) und zur Bewegung in Längsrichtung über den Schaft (22) ausgelegt ist;
worin jeder Dilatationskopf (26a, 26b, 26c) in dem Satz (122) von Dilatationsköpfen einen unterschiedlichen Außendurchmesser aufweist.

16. Instrumentensystem nach Anspruch 15, worin der Kolben (24) zwei längs angeordnete Stangen (56, 58) umfasst, die seitlich durch den Schaft (22) im Abstand gehalten werden, um eine Eingriffsspannung auf jeden mit dem distalen Ende (52) des Kolbens (24) verbundenen Dilatationskopf aufzubringen.

17. Instrumentensystem nach Anspruch 16, worin jede Stange (56, 58) einen Ansatz (82) umfasst, der von einer Außenseite der Stange (56, 58) vorsteht, und worin jeder Dilatationskopf eine Vertiefung (74) aufweist, die auf einer Innenseite des Dilatationskopfes geformt ist, wobei der Ansatz (82) zur Verbindung mit der Vertiefung (74) ausgelegt ist.

18. Instrumentensystem nach Anspruch 17, worin die Stangen (56, 58) seitlich bewegt werden können, um den Ansatz (82) aus seinem Eingriff mit der Vertiefung (74) zu lösen.

19. Instrumentensystem nach Anspruch 18, worin der Kolben (24) länger als der Schaft (22) ist, so dass sich die Stangen (56, 58) seitlich zusammen bewegen, wen sich ein distales Ende (52) des Kolbens (24) über ein distales Ende (32) des Schaftes (22) erstreckt.

20. Instrumentensystem nach Anspruch 1, ferner umfassend:
Mittle zur Entfernung des Dilatationskopfs von dem distalen Ende des Kolbens.

21. Instrumentensystem nach Anspruch 1, worin der Schaft (22) einen einzelnen monolithischen Schaft umfasst.

## Revendications

1. Instrument (20) configuré pour préparer un pénis pour l'implantation d'une prothèse pénienne, l'instrument (20) comportant :
un arbre (22) s'étendant entre une extrémité (32) distale et une extrémité (34) proximale ;
un plongeur (24) accouplé à l'arbre (22) et mobile par rapport à ce dernier ; et
une tête (26) de dilatation accouplée à une extrémité (52) distale du plongeur (24) et qui est disposée autour de l'extérieur (30) de l'arbre, **caractérisé en ce que** la tête de dilatation (26) est mobile longitudinalement le long de l'arbre (22).

2. Instrument selon la revendication 1, dans lequel le plongeur (24) comporte une première tige (56) et une seconde tige (58) et une surface (30) extérieure de l'arbre (22) délimite un premier canal (36) dimensionné pour recevoir la première tige (56) et un second canal (38) dimensionné pour recevoir la seconde tige (58).

3. Instrument selon la revendication 2, dans lequel le premier canal (36) et le second canal (38) sont des canaux ouverts de telle sorte qu'au moins une partie de la première tige (56) et de la seconde tige (58) est exposée à travers la surface (30) extérieure de l'arbre (22).

4. Instrument selon la revendication 2 ou 3, dans lequel le premier canal (36) est espacé d'une première distance (H1) par rapport au second canal (38) de telle sorte que les première et seconde tiges (56, 58) sont espacées de la première distance (H1) lorsqu'elles sont mises en prise dans les premier et second canaux (36, 38) respectifs.

5. Instrument selon la revendication 4, dans lequel les extrémités (52) distales des première et seconde tiges (56, 58) s'infléchissent latéralement de telle sorte que chacune est espacée de l'autre d'une distance (H2) qui est inférieure à la première distance (H1) lorsque l'extrémité (52) distale du plongeur (24) est déplacée au-delà de l'extrémité (32) distale de l'arbre (22).

6. Instrument selon la revendication 5, dans lequel les extrémités (52) distales des première et seconde tiges (56, 58) fournissent un verrou (80) fonctionnant sous tension, le verrou (80) fonctionnant sous tension se mettant en prise dans la tête (26) de dilatation lorsque l'extrémité (52) distale du plongeur (24) est située entre l'extrémité (32) distale et l'extrémité (34) proximale de l'arbre (22) et le verrou (80) fonctionnant sous tension étant désengagé de la tête (26) de dilatation lorsque l'extrémité (52) distale du plongeur (24) est déplacée au-delà de l'extrémité (32) distale de l'arbre (22).

7. Instrument selon l'une quelconque des revendications 1 à 6, dans lequel le plongeur (24) est plus long que l'arbre (22) et la tête (26) de dilatation est accouplée de manière détachable à une extrémité (52) distale du plongeur (24).

8. Instrument selon la revendication 7, comportant en outre :
une première poignée (40) fixée à l'extrémité (32) proximale de l'arbre (22) ; et
une seconde poignée (60) fixée à une extrémité (52) proximale du plongeur (24).

9. Instrument selon la revendication 8, dans lequel la première poignée (40) est séparée de la seconde poignée (60) lorsque l'extrémité (52) distale du plongeur (24) est située entre l'extrémité (32) distale et l'extrémité (34) proximale de l'arbre (22) et la première poignée (40) vient en contact avec la seconde poignée (60) lorsque l'extrémité (52) distale du plongeur (24) est déplacée au-delà de l'extrémité (32) distale de l'arbre (22).

10. Instrument selon l'une quelconque des revendications 1 à 9, dans lequel la tête (26) de dilatation est mobile de manière réversible longitudinalement entre l'extrémité (32) distale de l'arbre (22) et l'extrémité (34) proximale de l'arbre (22).

11. Instrument selon l'une quelconque des revendications précédentes 1 à 10, dans lequel une surface (30) extérieure de l'arbre (22) comporte des repères (42) de mesure.

12. Instrument selon l'une quelconque des revendications 1 à 11, dans lequel l'arbre (22) a un diamètre extérieur et la tête (26) de dilatation a un premier diamètre (D1) qui est supérieur au diamètre extérieur de l'arbre (22).

13. Instrument selon l'une quelconque des revendications 1 à 12, dans lequel l'arbre (22) a un diamètre extérieur constant.

14. Instrument selon l'une quelconque des revendications 1 à 13, dans lequel la tête (26) de dilatation est une première tête de dilatation qui est détachable du plongeur (24) et remplaçable par une seconde tête de dilatation qui a un second diamètre différent du premier diamètre de la première tête de dilatation.

15. Système d'instruments configuré pour préparer un pénis à recevoir une prothèse pénienne, le système d'instruments comportant un instrument (20) selon la revendication 1 et **caractérisé en outre par**
un arbre (22) longitudinal qui a un diamètre extérieur constant configuré pour mesurer l'ouverture dans un pénis ;
un plongeur (24) accouplé à l'arbre (22) et mobile longitudinalement dans ce dernier ; et
un ensemble (122) de têtes (26a, 26b, 26c) de dilatation, chaque tête de dilatation étant configurée pour s'accoupler à une extrémité (52) distale du plongeur (24), entourer l'extérieur (30) de l'arbre (22), et se déplacer longitudinalement le long de l'arbre (22) ;
dans lequel chaque tête (26a, 26b, 26c) de dilatation dans l'ensemble (122) de têtes de dilatation a un diamètre extérieur différent.

16. Système d'instruments selon la revendication 15, dans lequel le plongeur (24) comporte deux tiges (56, 58) disposées longitudinalement qui sont séparées latéralement l'une de l'autre par l'arbre (22) afin d'appliquer une tension de mise en prise à chaque tête de dilatation accouplée à l'extrémité (52) distale du plongeur (24).

17. Système d'instruments selon la revendication 16, dans lequel chaque tige (56, 58) comporte un bossage (82) qui fait saillie depuis une surface extérieure de la tige (56, 58), et chaque tête de dilatation comprend un renfoncement (74) formé sur une surface intérieure de la tête de dilatation, le bossage (82) étant configuré pour s'accoupler avec le renfoncement (74).

18. Système d'instruments selon la revendication 17, dans lequel les tiges (56, 58) sont mobiles latéralement pour désengager le bossage (82) du renfoncement (74).

19. Système d'instruments selon la revendication 18, dans lequel le plongeur (24) est plus long que l'arbre (22) de telle sorte que les tiges (56, 58) se déplacent latéralement ensemble lorsqu'une extrémité (52) distale du plongeur (24) s'étend sur une extrémité (32) distale de l'arbre (22).

20. Instrument selon la revendication 1, comportant en outre :
un moyen permettant d'enlever la tête de dilatation de l'extrémité distale du plongeur.

21. Instrument selon la revendication 1, dans lequel l'arbre (22) comporte un seul arbre monolithique.
